# EUROPEAN PATENT APPLICATION

(11) **EP 4 744 594 A1**
(43) Date of publication of application: **20.05.2026**
(21) Application number: 24212709.0
(22) Date of filing: 13.11.2024
(51) Int. Cl.: A61B 6/40, A61B 6/03, A61B 6/00

(54) **X-RAY IMAGING DEVICE AND METHOD FOR EXTENDING IMAGE PARAMETERS**

(71) Applicant: Bruker Belgium N.V., 2550 Kontich (BE)
(72) Inventor: Krumm, Michael, 2000 Antwerpen (BE); Bruyndonckx, Peter, 1980 Eppegem (BE)
(74) Representative: Altmann Stößel Dick Patentanwälte PartG mbB

(57) **Abstract**

The invention relates to a method for operating a computed tomography device (150), wherein the computed tomography device (150) comprises at least one x-ray radiation source (131) configured for generating x-ray radiation cones (132) for irradiating an object (134), wherein the computed tomography device (150) comprises at least one detector (136) configured for detecting the x-ray radiation of the x-ray radiation cones (132) that have interacted with the object (134), wherein the x-ray radiation source (131) comprises at least one electron optic arrangement (138) configured for variably arranging at least one electron beam (140) onto a plurality of positions of focal spots on at least one target comprised by the x-ray radiation source (131) in a manner that a plurality of different x-ray radiation cones (132) emerging from the focal spots is generated in order to image different portions of the object (134); the method comprising:
- performing a projection image scanning sequence (PSS) by using an imaging focal spot pattern in order to generate a plurality of projection images, wherein the imaging focal spot pattern comprises a plurality of x-ray focal spot positions on the at least one target (144) such that a volume of the object (134) to be reconstructed is irradiated by the different x-ray radiation cones (132);
- performing a tomographic scanning sequence (TSS) by performing the projection image scanning sequence (PSS) at differing viewing angles;
- performing an image reconstruction algorithm using the projection images in order to reconstruct a 3D-image of the object (134).

The invention further relates to a computed tomography device (150), a computer program and a computer-readable storage medium. The invention may be employed in order to extend the field of view for a specific viewing angle.

## Description

### Technical Field

The invention relates to a method for operating a computed tomography device, a computed tomography device, a computer program and a computer-readable storage medium.

The invention specifically may be used for imaging a living or non-living object. Specifically, the invention may offer an approach to extend the field of view by using a focal spot position pattern. Specifically, the focal spot position pattern may be selected with regard to the spatial extend of the object in all dimensions. Other fields of application of the present invention, however, are also feasible.

### Background art

In the field of x-ray imaging, the design of x-ray sources typically plays a major role and, thus, over the years, the design of x-ray sources has been dramatically improved. Major developments have, for example, focused on increasing the high voltage, improving a target power and a focal spot size as well as a stability resulting in a plurality of different X-ray source concepts. One way to differentiate between different x-ray source types may be a classification of the x-ray sources based on the focal spot size. There seems to be no rigid definition, clearly separating the different x-ray source types. However, the field is aware of macro-focus and mini-focus x-ray source types with focal spot sizes in the range of millimeters and sub-millimeters, respectively. Typically, the macro-focus x-ray sources may be closed-type evacuated x-ray sources. Micro-focus sources typically cover the range from 0.1 mm down to 1 µm focal spot size. These X-ray sources are typically open type x-ray sources that create vacuum during operation and that include x-ray optics to compress and shape the electron beam inside the electron gun of the x-ray source. Nowadays, there are also so-called nano-focus x-ray sources available that create focal spots in the sub-micron range down to 100-200 nm. To reach said small focal spots, it is necessary to have full control on the electron beam, the focusing units and the target design. Recent developments enable the feature to define the focal spot position on the target.

The patent application US 2021/410260 A1 discloses x-ray sources including an electron source, an adjustment means for adjusting an orientation of the electron beam generated by the electron source, a focusing means configured to focus the electron beam in accordance with a focusing setting, a beam orientation sensor arranged to generate a signal indicating an orientation of the electron beam relative to a target position, and a controller that is operably connected to the focusing means, the beam orientation sensor and the adjustment means. Also, X-ray sources including a target orientation sensor and a target adjustment means, wherein the controller is configured to cause the beam adjustment means and/or target adjustment means to adjust the relative orientation between the electron beam and the target are disclosed.

The patent application EP 3 240 011 A1 discloses technology for electronically aligning a central ray of an x-ray tube to a radiation detector. In an example, an x-ray system includes an x-ray tube and a tube control unit (TCU). The x-ray tube includes a cathode that includes an electron emitter configured to emit an electron beam, an anode configured to receive the electron beam and generate x-rays with a central ray from electrons of the electron beam colliding on a focal spot of the anode, and a steering magnetic multipole between the cathode and the anode that is configured to produce a steering magnetic field from a steering signal. At least two poles of the steering magnetic multipole are on opposite sides of the electron beam. The TCU includes at least one steering driver configured to generate the steering signal. The TCU is configured to convert an offset value to the steering signal.

Patent EP 3 764 325 B1 discloses a system according to an embodiment including circuitry. The circuitry inputs, to a first trained model third projection data to generate fourth projection data, receives the third projection data, the third projection data obtained from a CT (computed tomography) scan, and reconstructs a first image based on the fourth projection data. The first trained model has been trained using first projection data as an input and second projection data or first subtraction data between the second projection data and the first projection data as an output, the first projection data being acquired using an X-ray source having a first focal spot size, the second projection data being acquired using an X-ray source having a second focal spot size that is smaller than the first focal spot size, and the third projection data being acquired using an X-ray source having a third focal spot size that is larger than the second focal spot size.

The patent US 11 331 055 B2 discloses computed tomography apparatus and a method for influencing a position of a focal spot in an x-ray radiation source having a centering device to center an electron beam and a focus. The method includes positioning a reference object into a beam path of x-ray radiation between the x-ray radiation source and an x-ray radiation detector, the x-ray radiation detector having detector elements to generate an x-ray image, capturing an x-ray image of the reference object at different powers, reducing a focal spot displacement occurring at the different powers based on a comparison of the x-ray images captured at the different powers with one another, by setting at least one altered electric current to operate the centering device or the centering devices of the x-ray radiation source, and operating the computed tomography apparatus with the altered electric current, by which the focal spot displacement was reduced.

The international application WO 2012/ 123 843 A1 discloses an X-ray tube for stereoscopic imaging, an X- ray imaging system for stereoscopic imaging, a method for stereoscopic imaging as well as a computer program element and a computer readable medium for stereoscopic imaging. In order to provide stereoscopic imaging with improved space requirements and enhanced weight characteristics, an X-ray tube for stereoscopic imaging is provided, comprising a cathode, an anode, means for deflection of an electron beam, an X-ray aperture with at least a first hole and a second hole, and an envelope housing the cathode and the anode. The means for deflection are adapted such that the electron beam from the cathode can be deflected such that the electron beam hits the anode in at least two stereo focal spot positions separated from each other with a first distance defining a stereo direction. The aperture is fixedly arranged inside the envelope and an X-ray beam is generated at each focal spot emanating in a viewing direction through one of the at least first and second holes in the aperture. The direction of the first distance is perpendicular to the viewing direction, wherein the first distance is adaptable.

### Problem to be solved

It is therefore an object of the present invention to provide a method for operating a computed tomography device, a computed tomography device, a computer program and a computer-readable storage medium, which at least partially overcome the above-mentioned problems of the state of the art.

In particular, it is an object of the present invention to enlarge the field of view of a computed tomography device.

### Summary

This problem is addressed by a method for operating a computed tomography device and a computed tomography device, a computer program, a computer-readable storage medium for operating a computed tomography device in a manner to extend the field of view with the features of the independent claims. Advantageous embodiments which might be realized in an isolated fashion or in any arbitrary combinations are listed in the dependent claims as well as throughout the specification.

In a first aspect, a method for operating a computed tomography device is disclosed. For this aspect, reference may be made to any definition, embodiment and/or further aspect as disclosed elsewhere herein.

The method for operating a computed tomography device comprises the following steps, which may be performed in the given order. A different order, however, may also be feasible. Further, two or more of the method steps may be performed simultaneously. Thereby, the method steps may at least partly overlap in time. Further, the method steps may be performed once or repeatedly. Thus, one or more or even all of the method steps may be performed once or repeatedly. The method may comprise additional method steps, which are not listed herein.

The computed tomography device comprises at least one x-ray radiation source configured for generating x-ray radiation cones for irradiating an object. Further, the computed tomography device comprises at least one detector configured for detecting the x-ray radiation of the x-ray radiation cones that have interacted with the object. The x-ray radiation source comprises at least one electron optic arrangement configured for variably arranging at least one electron beam onto a plurality of positions of focal spots on at least one target comprised by the x-ray radiation source in a manner that a plurality of different x-ray radiation cones emerging from the focal spots is generated in order to image different portions of the object.

The term "computed tomography device" as used herein is a broad term and is to be given its ordinary and customary meaning to a person of ordinary skill in the art and is not to be limited to a special or customized meaning. The term specifically may refer, without limitation, to a device configured for performing an imaging technique in order to obtain 3D-image of an internal structure of an object non-destructively. The computed tomography (CT) device may be a micro-computed tomography configured to image the object at a micron-level resolution or a sub-micron level spatial resolution. The object may be a non-living object or a living object, such as an animal.

The term "x-ray radiation source" as used herein is a broad term and is to be given its ordinary and customary meaning to a person of ordinary skill in the art and is not to be limited to a special or customized meaning. The term specifically may refer, without limitation, to a device configured for emitting x-ray radiation. Specifically, the x-ray tube may comprise a cathode and a target, also referred to as anode. More specifically, the cathode may be a negative electrode and the target may be a positive electrode. The cathode may be configured to emit electrons, which may be accelerated to the target using a high voltage potential. The emitted electrons may also be referred to as electron beam. The electrons may hit the target at a certain position, thereby generating the x-ray radiation through bremsstrahlung. The spot where the electrons hit the target may also be referred to as focal spot.

The term "x-ray radiation cone" as used herein is a broad term and is to be given its ordinary and customary meaning to a person of ordinary skill in the art and is not to be limited to a special or customized meaning. The term specifically may refer, without limitation, to a plurality of x-ray beams, wherein the plurality of x-ray beams form a cone. The cone may be a three-dimensional geometric structure defined by a base, such as a rectangular, circular or elliptical base. Further base shapes are possible. Specifically, the cone tip or apex may emerge from the focal spot of the x-ray radiation source. The cone may expand from the tip or apex in a direction towards the detector. The shape of the x-ray radiation cone may be formed by using one or more collimators. Specifically, the collimator may be configured to absorb x-ray radiation. The x-ray radiation cone may be configured in a manner to irradiate at least one portion of the object, preferably in a manner that the x-ray radiation defining the x-ray radiation cone at least partially interacts with the object.

The term "electron optic arrangement" as used herein is a broad term and is to be given its ordinary and customary meaning to a person of ordinary skill in the art and is not to be limited to a special or customized meaning. The term specifically may refer, without limitation, to a device configured for adjusting the direction and/or the shape of the electron beam. Specifically, the electron optic arrangement may be configured to adjust the direction of the electron beam in the x-ray source such that the position of the focal spot on the target is adjusted. The electron optic arrangement may be configured to variably adjust the direction of the electron beam in the x-ray source such that the position of the focal spot on the target is variably adjusted. Specifically, the device may be or may comprise at least one magnet configured for adjusting the direction of the electron beam. More specifically, the device may be or may comprise at least one coil and/or deflector with a controllable and variable magnet field strength configured for variably adjusting the direction of the electron beam.

The term "focal spot position" as used herein is a broad term and is to be given its ordinary and customary meaning to a person of ordinary skill in the art and is not to be limited to a special or customized meaning. The term specifically may refer, without limitation, to a specific location of the focal spot on the target. Differing focal spot positions may generate differing x-ray radiation cones. The focal spot positions may differ for differing viewing angles.

The term "detecting", or any grammatical variation thereof, as used herein is a broad term and is to be given its ordinary and customary meaning to a person of ordinary skill in the art and is not to be limited to a special or customized meaning. The term specifically may refer, without limitation, to a quantitative and/or qualitative determination relating to the at least one property of an object, such as at least one of a physical, a chemical and a biological property, specifically a property such as an intensity of x-ray radiation or a number of photons. The term "detector" as used herein is a broad term and is to be given its ordinary and customary meaning to a person of ordinary skill in the art and is not to be limited to a special or customized meaning. The term specifically may refer, without limitation, to a device which is configured to detect at least one specific physical or chemical property. Specifically, the detector may be configured for detecting electromagnetic radiation. More specifically, the detector may be configured for detecting the intensity of incident x-rays or single x-ray photons. The detector may detect at least a portion of the x-ray radiation that has interacted with the object. Specifically, the detector may detect the intensity of the x-ray radiation that has interacted with the object. The detector may be a flat detector or a curved detector. The detector may be arranged in a manner that it detects x-ray radiation emerging from the x-ray focal spot positions.

The term "imaging", or any grammatical variation thereof, as used herein is a broad term and is to be given its ordinary and customary meaning to a person of ordinary skill in the art and is not to be limited to a special or customized meaning. The term specifically may refer, without limitation, to a process of generating at least one image of an object by performing a measurement. The image may be reconstructed from measurement data generated in the measurement. The measurement may be performed in order to detect at least one property of the object. Specifically, the term imaging may relate to irradiating the object with x-ray radiation, measuring the intensity of the incident x-ray radiation, and using this data to reconstruct an image of the object, preferably a 3D-image of the object.

The method comprises:
- performing a projection image scanning sequence (PSS) by using an imaging focal spot pattern in order to generate a plurality of projection images, wherein the imaging focal spot pattern comprises a plurality of x-ray focal spot positions on the at least one target such that a volume of the object to be reconstructed is irradiated by the different x-ray radiation cones;
- performing a tomographic scanning sequence (TSS) by performing the projection image scanning sequence (PSS) at differing viewing angles;
- performing an image reconstruction algorithm using the projection images in order to reconstruct a 3D-image of the object.

As already indicated, the method comprises performing a projection image scanning sequence (PSS) by using an imaging focal spot pattern in order to generate a plurality of projection images, wherein the imaging focal spot pattern comprises a plurality of x-ray focal spot positions on the at least one target such that a volume of the object to be reconstructed is irradiated by the different x-ray radiation cones.

The term "projection image" as used herein is a broad term and is to be given its ordinary and customary meaning to a person of ordinary skill in the art and is not to be limited to a special or customized meaning. The term specifically may refer, without limitation, to the measurement data received by the detector. In order to generate the projection image, the measurement data may be processed, such as by performing a logarithmic transformation of the measurement data or a normalization to a measurement without an object. A projection image may be recorded for a known focal spot position and a known viewing angle.

The term "projection image scanning sequence (PSS)" as used herein is a broad term and is to be given its ordinary and customary meaning to a person of ordinary skill in the art and is not to be limited to a special or customized meaning. The term specifically may refer, without limitation, to a sequence of a plurality of measurements of the object in order to generate measurement data. The measurement data may comprise a plurality of projection images, preferably recorded for differing focal spot positions. Specifically, PSS may refer to a sequence of measurements at one specific viewing angle, whereby for each measurement a different focal spot position is used in a manner that the plurality of projection images is recorded.

In the projection image scanning sequence, projection images may be recorded for a specific viewing angle. The differing x-ray radiation cones that are used to record the projections may not fully overlap within the object. Consequently, the differing x-ray radiation cones that are used to record the projections may at least be partially separated within the object. The projection images that may be recorded by doing so, consequently, show differing portions of the object.

The term "focal spot pattern" as used herein is a broad term and is to be given its ordinary and customary meaning to a person of ordinary skill in the art and is not to be limited to a special or customized meaning. The term specifically may refer, without limitation, to a specific arrangement of at least two focal spot positions for one specific viewing angle. By adjusting the focal spot positions as defined in the focal spot pattern, differing x-ray radiation cones may be generated. The differing x-ray radiation cones may irradiate differing portions of the volume of the object to be reconstructed. Thereby, the differing projection images may be generated. The "volume of the object to be reconstructed" may refer to a selected portion of the object for which said projection images are to be generated.

In other words, a plurality of projection images may be recorded for one specific viewing angle during the projection image scanning sequence (PSS). The plurality of projection images may be recorded for different focal spot positions. Thereby, the plurality of projection images may show a differing portion of the object.

As already indicated, the method comprises performing a tomographic scanning sequence (TSS) by performing the projection image scanning sequence (PSS) at differing viewing angles.

The term "tomographic scanning sequence (TSS)" as used herein is a broad term and is to be given its ordinary and customary meaning to a person of ordinary skill in the art and is not to be limited to a special or customized meaning. The term specifically may refer, without limitation, to a sequence of a plurality of measurements of the object in order to generate measurement data. The measurement data may comprise several pluralities of projection images recorded for differing viewing angles. The viewing angles, at which a projection image is recorded, may vary for differing TSS procedure. For a specific TSS procedure, a single projection image may be recorded. In this case, the viewing angle remains constant. In said TSS procedure, no 3D information on the object may be generated. In a further specific TSS procedure, the viewing angles may rotate through 360°, allowing complete 3D information to be captured from all perspectives around the object.

During the TSS a plurality of projection images of the object may be acquired. The projection images of the object may be captured across a range of viewing angles spanning more than 0°, preferably more than or equal to 180°, more preferably more than or equal to 360°. In order to do so, the object or the x-ray radiation source together with the detector may, either continuously or in small steps, rotate. During the TSS and the PSS, the relative arrangement between the x-ray radiation source and the detector preferably may remain fixed. During the TSS and the PSS, the detector and the x-ray radiation source preferably may have a fixed position on the gantry, and preferably may be not laterally movable.

The term "viewing angle" as used herein is a broad term and is to be given its ordinary and customary meaning to a person of ordinary skill in the art and is not to be limited to a special or customized meaning. The term specifically may refer, without limitation, to the orientation or rotational alignment of the detector relative to the object being imaged. A specific viewing angle may refer to a specific angular position of the x-ray radiation source in relation to the object. In order to adjust the viewing angle, the x-ray radiation source and the detector may rotate together around the object. The orientation and/or position of the object may remain during said rotation. Alternatively, or in addition, the object may be rotated while the x-ray radiation source and the detector remain in their positions.

Specifically, TSS may refer to a sequence of measurements performed at different viewing angles. More specifically, TSS may refer to a sequence of measurements, whereby at each specific viewing angle at least one PSS is performed. The focal spot pattern may comprise a plurality of known specific arrangements of at least two focal spot positions for each specific viewing angle.

Several pluralities of projection images may be recorded for differing specific viewing angles during the tomographic scanning sequence (TSS). Thereby, the plurality of projection images may show projections of the volume of the object to be reconstructed from differing viewing angles.

As already indicated, the method comprises performing an image reconstruction algorithm using the projection images in order to reconstruct a 3D-image of the object.

The term "reconstructing" as used herein is a broad term and is to be given its ordinary and customary meaning to a person of ordinary skill in the art and is not to be limited to a special or customized meaning. The term specifically may refer, without limitation, to the process of calculating an image by evaluating measurement data.

Determining the 3D-image of the object by using the image reconstruction algorithm may comprise evaluating each captured projection image and, for each projection image, using the x-ray focal spot position on the at least one target at which the respective projection image has been captured. Alternatively or in addition, the method may further comprise determining the 3D-image of the object by using the image reconstruction algorithm by evaluating each captured projection image and, for each projection image, using the viewing angle at which the respective projection image has been captured. In other words, in order to generate the 3D image a plurality of projection images may be evaluated, preferably by using the image reconstruction algorithm, particularly by using a known focal spot position and a known viewing angle at which a specific projection image of the plurality of projection images is recorded.

Specifically, the term reconstructing may comprise using a reconstruction algorithm configured to determine the image from measurement data. The term "image reconstructing algorithm" as used herein is a broad term and is to be given its ordinary and customary meaning to a person of ordinary skill in the art and is not to be limited to a special or customized meaning. The term specifically may refer, without limitation, to a defined procedure of reconstructing an image, specifically the 3D image, by evaluating the recorded measurement data.

The image reconstruction algorithm may be or may comprise at least one iterative reconstruction algorithm. The term "iterative reconstruction algorithm" as used herein is a broad term and is to be given its ordinary and customary meaning to a person of ordinary skill in the art and is not to be limited to a special or customized meaning. The term specifically may refer, without limitation, to a computational method used to progressively refine an estimate of a solution. The iterative reconstruction algorithm may repeatedly adjust the solution by applying a set of rules or corrections based on a comparison between the current estimate and the observed data or constraints.

Further, the image reconstruction algorithm may be or may comprise at least one analytical reconstruction algorithm. The term "analytical reconstruction algorithm" as used herein is a broad term and is to be given its ordinary and customary meaning to a person of ordinary skill in the art and is not to be limited to a special or customized meaning. The term specifically may refer, without limitation, to a computational method used to determine a solution by applying one or more rules. The analytical reconstruction algorithm may be used to directly derive the solution by using the one or more rules.

The analytical reconstruction algorithm may be or may comprise a Feldkamp (FDK)-type algorithm. During the back projection, the contribution of each projection image recorded at a given focal spot position to a given image pixel may be weighted by a factor that scales inversely with the contribution of the corresponding x-ray radiation cones to that pixel. The sum of the different x-ray radiation cone weights for a given pixel may be unity.

Performing a tomographic scanning sequence (TSS) by performing the projection image scanning sequence (PSS) at differing viewing angles may either be done by
- performing a rotation of the x-ray source - detector - assembly relative to the object and generating a projection image for a specific focal spot position comprised by the imaging focal spot pattern, and repeating this procedure by performing at least one further rotation until the projection images have been recorded for each specific focal spot position comprised by the imaging focal spot pattern; or
- performing a sub rotation of the x-ray source - detector - assembly relative to the object and generating projection images for each specific focal spot position comprised by the imaging focal spot, and repeating this procedure by performing at least one further sub rotation until the projection images have been recorded for each sub rotation comprised by the imaging focal spot pattern.

The rotation may be associated with viewing angles spanning more than 0°, preferably more than or equal to 180°, more preferably more than or equal to 360°.

The term "3D image" as used herein is a broad term and is to be given its ordinary and customary meaning to a person of ordinary skill in the art and is not to be limited to a special or customized meaning. The term specifically may refer, without limitation, to an image resulting after reconstruction of measurement data which expands in three dimensions. More specifically, 3D image refers to a three-dimensional representation of the volume of the object to be reconstructed, typically, generated by evaluating the projection images.

The imaging focal spot pattern may be variable in a manner that the x-ray focal spot positions on the at least one target are variable. The term "variable" as used herein is a broad term and is to be given its ordinary and customary meaning to a person of ordinary skill in the art and is not to be limited to a special or customized meaning. The term specifically may refer, without limitation, to the possibility to change or vary a value that can be modified. The x-ray focal spot positions may be variable, such that the generated x-ray cones may irradiate a variable portion of the volume of the object to be reconstructed.

The method may further comprise determining the variable x-ray focal spot positions on the at least one target by using a known 3D spatial extend of the object. The known 3D spatial extend may, thereby, be approximated by using a bounding box enclosing the object. The variable x-ray focal spot positions may be determined in order to increase the field of view of the detector to cover the complete volume of the object to be reconstructed. The x-ray focal spot positions may be determined such that the entire volume of the object to be reconstructed is completely exposed by the emerging x-ray radiation cones at each of the viewing angles.

The term "3D spatial extend of an object" as used herein is a broad term and is to be given its ordinary and customary meaning to a person of ordinary skill in the art and is not to be limited to a special or customized meaning. The term specifically may refer, without limitation, to the dimension of at least a portion of the object, specifically of the volume of the object to be reconstructed, in all three space coordinates. The known 3D spatial extend of the object may be evaluated in order to select specific x-ray focal spot positions in a manner that the generated x-ray radiation irradiates the volume of the object to be reconstructed such that corresponding projection images may be recorded. Thereby, the field of view of the detector is adjusted in a manner that the volume of the object to be reconstructed is imaged. The term "field of view" as used herein is a broad term and is to be given its ordinary and customary meaning to a person of ordinary skill in the art and is not to be limited to a special or customized meaning. The term specifically may refer, without limitation, to the spatial extend of the object that are exposed to x-rays and are contained within the x-ray radiation cones and that is captured by the detector.

In order to reconstruct the 3D image of the object, said volume of the object to be reconstructed may have to be included in the field of view of the detector. However, the 3D spatial extend of the object may extend the field of view of the detector for a specific focal spot position and corresponding x-ray radiation cone. Thus, varying the focal spot position may increase the field of view such that the entire volume of the object to be reconstructed is captured, preferably for a specific viewing angle.

The term "bounding box" as used herein is a broad term and is to be given its ordinary and customary meaning to a person of ordinary skill in the art and is not to be limited to a special or customized meaning. The term specifically may refer, without limitation, to a geometric frame encapsulating at least a portion of the object, in a manner that the 3D spatial extend of an object is approximated. The 3D spatial extend of an object may particularly be approximated in the volume of the object to be reproduced and/or the entire object. Specifically, the bounding box may approximate the geometric form of the object by using a simpler geometric form. More specifically, the coordinates of the bounding box defining the geometry of the bounding box may be selected such that at least the portion of the object lies within the bounding box.

The method may further comprise:
- determining the known 3D spatial extend of the object by at least one of:
   ∘ performing a scouting procedure;
   ∘ receiving a user-input related to the known 3D spatial extend of the object;
   ∘ receiving data related to the 3D spatial extend of the object, wherein the data related to the 3D spatial extend of the object is selected from at least one of:
      ▪ data of a model of the object; such as a CAD model of the object;
      ▪ data of a 3D volume representation of the object, such as existing 3D tomographic data or volumetric data;

The determined known 3D spatial extend of the object may be an approximated 3D spatial extend of the object. Particularly, the approximated 3D spatial extend of the object may comprise at least one simplified geometric object, such as a cube, a cylinder, a sphere, a cone.

Determining the known 3D spatial extend of the object may be performed as an initial step of the method for operating a computed tomography device, such that the imaging focal spot pattern can be determined.

The term "scouting procedure" as used herein is a broad term and is to be given its ordinary and customary meaning to a person of ordinary skill in the art and is not to be limited to a special or customized meaning. The term specifically may refer, without limitation, to a process of performing at least one radiographic measurement in order to determine the 2D and/or 3D spatial extend of the object.

The term "user input" as used herein is a broad term and is to be given its ordinary and customary meaning to a person of ordinary skill in the art and is not to be limited to a special or customized meaning. The term specifically may refer, without limitation, to a user generated information that is received by the computed tomography device. The user input may be received via a connection interface and/or a user input module comprised by the computed tomography device. Therefore, the user may enter the user input into the user input module, such as by text and/or voice and/or by a selection of a graphical control element. More specifically, the term user input may refer to information about the 3D spatial extend of the object, such as a height, a length, a width or similar related to the 3D spatial extend of the object. The user input may describe the 3D spatial extend of the object by using geometric objects, such as a cube, a cylinder, a sphere, a cone, and respective dimensions that are selected to approximate the 3D spatial extend of the object.

The term "CAD model" as used herein is a broad term and is to be given its ordinary and customary meaning to a person of ordinary skill in the art and is not to be limited to a special or customized meaning. The term specifically may refer, without limitation, to a geometrical model of an object which has been generated by using a Computer-Aided Design (CAD) software. Specifically, a CAD model may comprise data on the 3D spatial extend of an object.

The term "3D tomographic data" as used herein is a broad term and is to be given its ordinary and customary meaning to a person of ordinary skill in the art and is not to be limited to a special or customized meaning. The term specifically may refer, without limitation, to information comprised by at least one known projection image and/or at least one known 3D image, which has been recorded in a previous computed tomographic imaging scan.

The scouting procedure may comprise performing a PSS at a plurality of differing viewing angles. Therein, the PSS may use a scout scan pattern comprising one or more focal spot positions on the at least one target. The one or more x-ray focal spot positions on the at least one target may be selected in order to determine the edges of the object. The scouting procedure may further comprise constructing the bounding box around the object for each viewing angle of the plurality of differing viewing angles. The known 3D spatial extend of the object may be approximated by using the bounding box.

The term "scout scan pattern" as used herein is a broad term and is to be given its ordinary and customary meaning to a person of ordinary skill in the art and is not to be limited to a special or customized meaning. The term specifically may refer, without limitation, to a specific arrangement of at least two focal spot positions for one specific viewing angle. By adjusting the focal spot positions as defined in the scout spot pattern, differing x-ray radiation cones may be generated. The differing x-ray radiation cones may irradiate differing portions of the object. The x-ray cones that are generated for the differing focal spot positions encompass differing measuring fields that extend from the x-ray radiation source to the detector. The differing measuring fields define a combined measuring field. For the scout scan pattern to be selected in order to determine the edges of the object, the scout scan pattern may be selected in a manner that the combined measuring fields encompasses the object in a manner that the edges can be determined.

The computed tomography device may have a variable geometry, preferably such that a distance between the object and the x-ray radiation source and/or the distance between the object and the detector, may be variable.

The term "geometry of a CT device" as used herein is a broad term and is to be given its ordinary and customary meaning to a person of ordinary skill in the art and is not to be limited to a special or customized meaning. The term specifically may refer, without limitation, to a spatial arrangement of components comprised by the CT device. The components may be or may comprise at least one of the object; the x-ray source; the detector. The geometry of the CT device may be variable such that the arrangement of said components may be adjusted, specifically as part of the method for operating a computed tomography device. By adjusting the arrangement of said components, the distance between the object and the detector and/or the distance between the x-ray source and the detector and/or the distance between the x-ray source and the object may be adjusted.

The scouting procedure may further comprise adjusting the zoom of the computed tomography device. Preferably, the zoom may be adjusted by adjusting the distance between the object and the x-ray radiation source and/or adjusting the distance between the object and the detector, in a manner that the portion of the object covered by the x-ray cone increases.

Preferably, the portion of the object covered by the x-ray cone may increase such that a single projection image may completely capture the object so that only one focal spot scouting position may be required in order to determine the edges of the object and to construct the bounding box.

Typically, the object is arranged between the x-ray source and the detector. Consequently, the object may be arranged within a measurement field. The "measurement field" may be a volume encompassed by the x-ray radiation, wherein the detector may be sensitive to x-ray radiation within the volume. The term "zoom" as used herein is a broad term and is to be given its ordinary and customary meaning to a person of ordinary skill in the art and is not to be limited to a special or customized meaning. An x-ray cone that is generated for a specific focal spot position encompasses a measurement field that extends from the x-ray radiation source to the detector.

Specifically depending on the size of the object, the object may not be fully encompassed by said measurement field. Rather, only a portion of the object may be encompassed.

In order to adjust the zoom of the computed tomography device, the variable geometry of the CT device may be adjusted. The variable geometry may allow to adjust the position of at least one of: the object; the x-ray source; the detector within the CT device. Thereby, the portion of the object that is encompassed by said measurement field may be adjusted. Said measurement field may be adjusted in a manner that the portion of the object being encompassed by said measurement field is increased, preferably being against the constraints defined by the geometry of a CT device. As an example, the variable geometry of the CT device may be adjusted such that said measurement field encompasses the volume of the object to be reconstructed and/or the entire object. Thereby, the number a projection images that have to be generated in the scouting procedure may be minimized.

As already indicated, the method may comprise determining the variable x-ray focal spot positions on the at least one target by using a known 3D spatial extend of the object. Said x-ray focal spot positions may be absolute x-ray focal spot positions. The determining may require evaluating the known 3D spatial extend of the object and the relative positions of the x-ray radiation source, the object and/or the detector.

The focal spot pattern may be selected in a manner that the complete object volume that needs to be reconstructed is fully irradiated. In other words, each part of the volume to be reconstructed may be irradiated by an x-ray cone beam associated with at least one focal spot comprised by the focal spot pattern. This constraint, based on the known 3D spatial extend of the object and the relative positions of the x-ray radiation source, the object and/or the detector may define, which focal spot patterns are possible.

The method may further comprise determining a maximum distance between, specifically neighboring, x-ray focal spot positions on the at least one target in the imaging focal spot pattern such that the complete volume of the object to be reconstructed may be irradiated by the different x-ray radiation cones when a PSS is performed. The maximum distance may be constant between any pair of neighboring x-ray focal spot positions comprised by the imaging focal spot pattern. Therein, determining a maximum distance may be performed by evaluating the known 3D spatial extend of the object and the relative positions of the x-ray radiation source, the object and/or the detector.

The distance between the object and the x-ray radiation source may define the portion of the object that is irradiated. The maximum distance between x-ray focal spot positions may be determined in a manner that
- the overlap between the portions of the object, which are irradiated by the different x-ray radiation cones, is minimized; and that
- the complete volume of the object to be reconstructed is irradiated.

By determining the maximum distance between the x-ray focal spot positions on the at least one target in the imaging focal spot pattern, the relative arrangement of the x-ray focal spot position on the target may be determined.

The term "maximum distance between neighboring x-ray focal spot positions" as used herein is a broad term and is to be given its ordinary and customary meaning to a person of ordinary skill in the art and is not to be limited to a special or customized meaning. The term specifically may refer, without limitation, to a spatial relationship between at least focal spot positions. The maximum distance between neighboring x-ray focal spot positions may refer to physical distance between two specific focal spot positions for which the portions of the object that are irradiated by the corresponding x-ray radiation cones are in contact with each other and have an immediate connection between their geometrical centers without a further x-ray radiation cone related to a further x-ray focal spot position in between.

The method may further comprise determining a minimum number of x-ray focal spot positions on the at least one target required in the imaging focal spot pattern such that the complete object volume to be reconstructed is irradiated when a PSS is performed. Therein, determining a minimum number of x-ray focal spot positions may be performed by evaluating the known 3D spatial extend of the object and the relative positions of the x-ray radiation source, the object and/or the detector.

Further, the determination of the maximum distance between the neighboring x-ray focal spot positions on the at least one target in the imaging focal spot pattern may be used to determine the minimum number of x-ray focal spot positions on the at least one target. Therefore, the relative arrangement of the x-ray focal spot position on the target may be aligned on the target so that the absolute arrangement of the x-ray focal spot position on the target is known. Thereby, the absolute focal spot position in the imaging focal spot pattern may be determined.

Alternatively, the minimum number of x-ray focal spot positions may be determined directly without determining the maximum distance between the x-ray focal spot positions, such as by trial and error and/or by performing a regression algorithm.

The maximum distance between x-ray focal spot positions on the at least one target in the imaging focal spot pattern and the minimum number of x-ray focal spot positions in the imaging focal spot pattern may be determined for each viewing angle of the differing viewing angles of the TSS. Consequently, the absolute focal spot positions in the imaging focal spot pattern may be determined for each viewing angle. The absolute focal spot positions in the imaging focal spot pattern may be differing for each viewing angle.

The imaging focal spot pattern may degenerate into a horizontal line pattern in case only a trans-axial field of view of the detector needs to be extended. Alternatively, the imaging focal spot pattern may degenerate into a vertical line pattern in case only an axial field of view of the detector needs to be increased. A superposition may further be possible.

In a further aspect, a computed tomography device is disclosed. Therein, the computed tomography device comprises at least one x-ray radiation source configured for generating x-ray radiation cones for irradiating an object. Further, the computed tomography device comprises at least one detector configured for detecting the x-ray radiation of the x-ray radiation cones that have interacted with the object. The x-ray radiation source comprises at least one electron optic arrangement configured for variably arranging at least one electron beam onto a plurality of positions of focal spots on at least one target comprised by the x-ray radiation source in a manner that a plurality of different x-ray radiation cones emerging from the focal spots is generated in order to image different portions of the object. The computed tomography device is configured for performing the method as disclosed elsewhere herein. For this aspect, reference may be made to any disclosure herein, particularly to any definition, Embodiment and/or further aspect as disclosed elsewhere herein.

In a further aspect, a computer program is disclosed. The computer program comprises instructions which, when the instructions are executed by the computed tomography device as disclosed elsewhere herein, cause the computed tomography device to perform the method as disclosed elsewhere herein. For this aspect, reference may be made to any disclosure herein, particularly to any definition, Embodiment and/or further aspect as disclosed elsewhere herein.

In a further aspect, a computer-readable storage medium is disclosed. Specifically, a non-transient computer-readable medium is disclosed, comprising instructions which, when the instructions are executed by the computed tomography device as disclosed elsewhere herein, cause the computed tomography device to perform the method as disclosed elsewhere herein. For this aspect, reference may be made to any disclosure herein, particularly to any definition, Embodiment and/or further aspect as disclosed elsewhere herein.

As used herein, the term "computer-readable storage medium" specifically may refer to non-transitory data storage means, such as a hardware storage medium having stored thereon computer-executable instructions. The computer-readable storage medium specifically may be or may comprise a storage medium such as a random-access memory (RAM) and/or a read-only memory (ROM).

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The proposed method for operating a computed tomography device, the computed tomography device, the computer program and the computer-readable storage medium may provide many advantages over known methods and devices.

Typically, the field of view of a detector is limited by the size of the detector, i.e. the active area. In case the field view is too small for fully projecting the object onto the detector, the state of the art is to move the detector or the object, specifically relative to the x-ray radiation source, to achieve a different perspective onto the object that allows to extract additional information of objection regions that are invisible for a single field of view. Typically, the detector is moved within the gantry. The detector may be moved by an offset value leaving an overlap between the viewing angels such that the recorded projection images may be stitched to a common projection image. In fact, even one of the recorded projection images may be sufficient to reconstruct the CT image completely as long as the viewing angle covers full 360°. Tuy's condition may guarantee that every point of the CT image has been "seen" from at least 180°.

The disadvantage of this method may be that a physical movement of the detector is required, which may be time consuming and may be prone to mechanical inaccuracies. Instead, with the proposed method here, the focal spot is positioned on the target of the x-ray radiation source such that it is created in a slightly different position resulting in a different scanning geometry. The different scanning geometry may allow for the collection of the relevant data such that all information can be collected as if the detector would have been moved. It may remain part of the 3D image reconstruction to ensure that the individual projection images are used correctly with respect to in the actual scanning geometry.

The disclosure aims at replacing the mechanical movement of a detector with displacements of the x-ray focal spot position to enlarge the field of view of x-ray imaging systems. The x-ray focal spot adjustment may be achieved by electron beam optics in the x-ray radiation source, eliminating the necessity for slow and possible inaccurate mechanical movements of the detector. Moving the focal spot position allows different parts of the object volume to be sampled, which may otherwise not fit into a single cone-beam projection onto a detector. The x-ray focal spot may be moved in steps until the complete object volume has been covered by the plurality of cone beams. If the x-ray focal spot may be moved in 2D, both the radial and axial field of view may be enlarged.

Contrary to the FOV enlargement method using a mechanical movement of the detector, the focal spot movement method may result in a significant overlap of the x-ray radiation cones at the different focal spot positions. This may lead to an oversampling of the object in comparison to a system with a stationary x-ray focal spot and detector. The extra information due to the oversampling may be used during the image reconstruction process to improve a signal-to-noise ratio, a resolution and/or an image quality in general.

Summarizing and without excluding further possible embodiments, the following embodiments may be envisaged:
Embodiment 1: A method for operating a computed tomography device, wherein the computed tomography device comprises at least one x-ray radiation source configured for generating x-ray radiation cones for irradiating an object, wherein the computed tomography device comprises at least one detector configured for detecting the x-ray radiation of the x-ray radiation cones that have interacted with the object, wherein the x-ray radiation source comprises at least one electron optic arrangement configured for variably arranging at least one electron beam onto a plurality of positions of focal spots on at least one target comprised by the x-ray radiation source in a manner that a plurality of different x-ray radiation cones emerging from the focal spots is generated in order to image different portions of the object; the method comprising:
   - performing a projection image scanning sequence (PSS) by using an imaging focal spot pattern in order to generate a plurality of projection images, wherein the imaging focal spot pattern comprises a plurality of x-ray focal spot positions on the at least one target such that a volume of the object to be reconstructed is irradiated by the different x-ray radiation cones;
   - performing a tomographic scanning sequence (TSS) by performing the projection image scanning sequence (PSS) at differing viewing angles;
   - performing an image reconstruction algorithm using the projection images in order to reconstruct a 3D-image of the object.
Embodiment 2: The method according to the preceding embodiment, wherein the imaging focal spot pattern is variable in a manner that the x-ray focal spot positions on the at least one target are variable.
Embodiment 3: The method according to the preceding embodiment, further comprising:
   - determining the variable x-ray focal spot positions on the at least one target by using a known 3D spatial extend of the object, preferably wherein the known 3D spatial extend is approximated by using a bounding box enclosing the object, wherein the variable x-ray focal spot positions are determined in order to increase the field of view of the detector to cover the complete volume of the object to be reconstructed.
Embodiment 4: The method according to the preceding embodiment, further comprising:
   - determining the known 3D spatial extend of the object by at least one of:
      ∘ performing a scouting procedure;
      ∘ receiving a user-input related to the known 3D spatial extend of the object;
      ∘ receiving data related to the 3D spatial extend of the object, wherein the data related to the 3D spatial extend of the object is selected from at least one of:
         ▪ data of a model of the object; such as a CAD model of the object;
         ▪ data of a 3D volume representation of the object, such as existing 3D tomographic data or volumetric data;
Embodiment 5: The method according to the preceding embodiment, wherein the determined known 3D spatial extend of the object is an approximated 3D spatial extend of the object, particularly wherein the approximated 3D spatial extend of the object comprises at least one simplified geometric object, such as a cube, a cylinder, a sphere, a cone.
Embodiment 6: The method according to the preceding embodiment, wherein the scouting procedure comprises
   - performing a PSS at a plurality of differing viewing angles, wherein the PSS is using a scout scan pattern comprising one or more focal spot positions on the at least one target, wherein the one or more x-ray focal spot positions on the at least one target are selected in order to determine the edges of the object.
Embodiment 7: The method according to the preceding embodiment, wherein the scouting procedure further comprises
   - constructing the bounding box around the object for each viewing angle of the plurality of differing viewing angles, wherein the known 3D spatial extend of the object is approximated by using the bounding box.
Embodiment 8: The method according to the preceding embodiment, wherein the computed tomography device has a variable geometry, preferably such that a distance between the object and the x-ray radiation source and/or the distance between the object and the detector, is variable, wherein the scouting procedure further comprises
   - adjusting the zoom of the computed tomography device, preferably by adjusting the distance between the object and the x-ray radiation source and/or adjusting the distance between the object and the detector, in a manner that the portion of the object covered by the x-ray cone increases, preferably such that a single projection image completely captures the object so that only one focal spot scouting position is required in order to determine the edges of the object and to construct the bounding box.
Embodiment 9: The method according to any one of the five preceding embodiments, further comprising:
   - determining a maximum distance between x-ray focal spot positions on the at least one target in the imaging focal spot pattern such that the complete volume of the object to be reconstructed is irradiated by the different x-ray radiation cones when a PSS is performed, wherein determining a maximum distance is performed by evaluating the known 3D spatial extend of the object and the relative positions of the x-ray radiation source, the object and the detector.
Embodiment 10: The method according to any one of the six preceding embodiments, further comprising:
   - determining a minimum number of x-ray focal spot positions on the at least one target required in the imaging focal spot pattern such that the complete object volume to be reconstructed is irradiated when a PSS is performed, wherein determining a minimum number of x-ray focal spot positions is performed by evaluating the known 3D spatial extend of the object and the relative positions of the x-ray radiation source, the object and the detector.
Embodiment 11: The method according to the preceding embodiments, where the maximum distance between x-ray focal spot positions on the at least one target in the imaging focal spot pattern and the minimum number of x-ray focal spot positions in the imaging focal spot pattern is determined for each viewing angle of the differing viewing angles of the TSS.
Embodiment 12: The method according to any one of the preceding embodiments, where the imaging focal spot pattern degenerates into a horizontal line pattern in case only a trans-axial field of view of the detector needs to be extended; or where the imaging focal spot pattern degenerates into a vertical line pattern in case only an axial field of view of the detector needs to be increased.
Embodiment 13: The method according to any one of the preceding embodiments, wherein determining the 3D-image of the object by using the image reconstruction algorithm comprises evaluating each captured projection image and, for each projection image, using the x-ray focal spot position on the at least one target at which the respective projection image has been captured.
Embodiment 14: The method according to the preceding embodiment, wherein the image reconstruction algorithm is or comprises at least one iterative reconstruction algorithm.
Embodiment 15: The method according to any one of the two preceding embodiments, wherein the image reconstruction algorithm is or comprises at least one analytical reconstruction algorithm.
Embodiment 16: A computed tomography device, wherein the computed tomography device comprises at least one x-ray radiation source configured for generating x-ray radiation cones for irradiating an object, wherein the computed tomography device comprises at least one detector configured for detecting the x-ray radiation of the x-ray radiation cones that have interacted with the object, wherein the x-ray radiation source comprises at least one electron optic arrangement configured for variably arranging at least one electron beam onto a plurality of positions of focal spots on at least one target comprised by the x-ray radiation source in a manner that a plurality of different x-ray radiation cones emerging from the focal spots is generated in order to image different portions of the object, wherein the computed tomography device is configured for performing the method according to any one of the preceding embodiments.
Embodiment 17: A computer program comprising instructions which, when the instructions are executed by the computed tomography device according to the embodiment referring to the computed tomography device, cause the computed tomography device to perform the method according to any one of the preceding claims referring to a method.
Embodiment 18: A computer-readable storage medium, specifically a non-transient computer-readable medium, comprising instructions which, when the instructions are executed by the computed tomography device according to the embodiment referring to the computed tomography device, cause the computed tomography device to perform the method according to any one of the preceding claims referring to a method.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

### In the Figures:

- Figure 1: shows an exemplary computed tomography device;
- Figure 2: shows an exemplary method for operating a computed tomography device; and
- Figure 3: shows a further exemplary computed tomography device.

### Detailed description of the embodiments

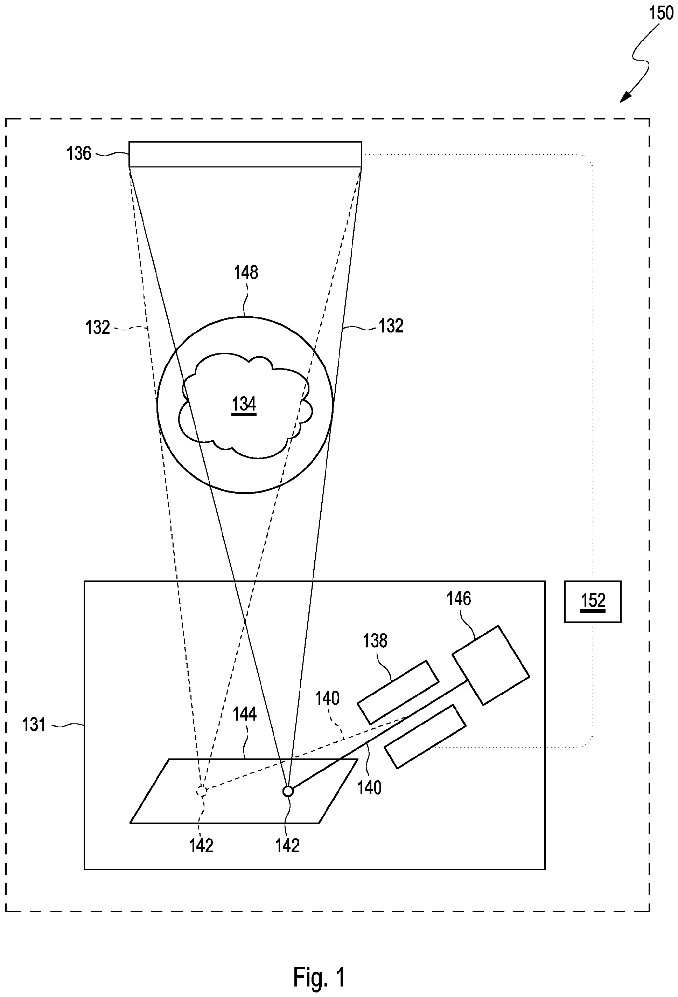

In Figure 1, an exemplary computed tomography device 150 is shown. Therein, the computed tomography device comprises at least one x-ray radiation source 131 configured for generating x-ray radiation cones 132 for irradiating an object 134. Further, the computed tomography device comprises at least one detector 136 configured for detecting the x-ray radiation of the x-ray radiation cones 132 that have interacted with the object 134.

The x-ray radiation source 131 comprises at least one electron optic arrangement 138 configured for variably arranging at least one electron beam 140 onto a plurality of positions of focal spots 142 on at least one target 144 comprised by the x-ray radiation source 131 in a manner that a plurality of different x-ray radiation cones 136 emerging from the focal spots 142 is generated in order to image different portions of the object 134. The electron beam 140 is, typically, generated by using an electron gun 146 comprised by the x-ray radiation source 131.

The computed tomography device 150 may comprise a control unit 152 which may be operatively connected to the at least one electron optic arrangement 138 to adjust the focal spot position of the electron beam 140 on the target 144. Further, the control unit 152 may be operatively connected to the detector 136.

Further, in Figure 1, two exemplary trajectories of the electron beam 140 are shown in a solid and a dashed line style. The two exemplary emerging x-ray radiation cones 136 encompass the entire cross-section of the object 134, which represents part of the volume of the object 134 to be reconstructed. The object 134 may be approximated by a bounding box 148, as shown exemplarily, but not limiting, in Figure 1. By doing so, the field of view of the detector 136 is increased, whereby the relative arrangement between the detector 136 and the x-ray radiation source 131 is maintained.

The computed tomography device 150 is configured for performing a method 110 for operating a computed tomography device 150.

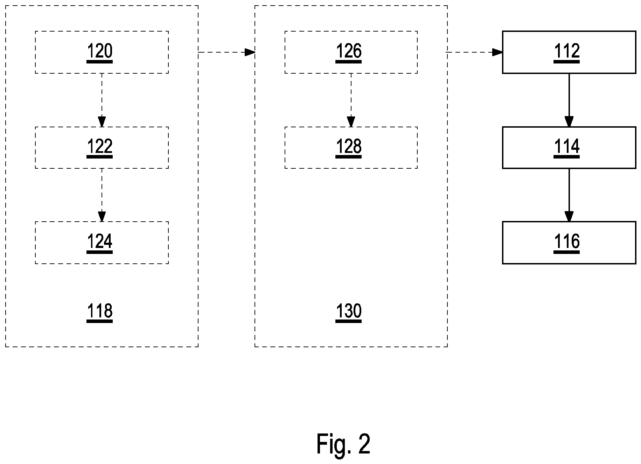

In Figure 2, an exemplary method 110 for operating a computed tomography device 150 is shown. The method comprises:
- (denoted by reference number 112) performing a projection image scanning sequence (PSS) by using an imaging focal spot pattern in order to generate a plurality of projection images, wherein the imaging focal spot pattern comprises a plurality of x-ray focal spot positions on the at least one target 144 such that a volume of the object 134 to be reconstructed is irradiated by the different x-ray radiation cones 132;
- (denoted by reference number 114) performing a tomographic scanning sequence (TSS) by performing the projection image scanning sequence (PSS) at differing viewing angles;
- (denoted by reference number 116) performing an image reconstruction algorithm using the projection images in order to reconstruct a 3D-image of the object.

The steps 112 and 114 may be repeated in order to increase the number of projection images such that the resolution of the 3D image increases as a result of providing additional projection images of the object.

The imaging focal spot pattern may be variable in a manner that the x-ray focal spot positions on the at least one target are variable.

The method may further comprise determining the variable x-ray focal spot positions (denoted by reference number 130) on the at least one target 144 by using a known 3D spatial extend of the object. Preferably, the known 3D spatial extend may be approximated by using a bounding box 148 enclosing the object 134, wherein the variable x-ray focal spot positions may be determined in order to increase the field of view of the detector 136 to cover the complete volume of the object 134 to be reconstructed.

The method may further comprise:
- (denoted by reference number 118) determining the known 3D spatial extend of the object 134 by at least one of:
   ∘ performing a scouting procedure;
   ∘ receiving a user-input related to the known 3D spatial extend of the object;
   ∘ receiving data related to the 3D spatial extend of the object, wherein the data related to the 3D spatial extend of the object is selected from at least one of:
      ▪ data of a model of the object; such as a CAD model of the object 134;
      ▪ data of a 3D volume representation of the object, such as existing 3D tomographic data or volumetric data;

The determined known 3D spatial extend of the object may be an approximated 3D spatial extend of the object. Particularly, the approximated 3D spatial extend of the object may comprise at least one simplified geometric object, such as a cube, a cylinder, a sphere, a cone.

The scouting procedure may comprise performing a PSS at a plurality of differing viewing angles (denoted by reference number 120). Therein, the PSS may use a scout scan pattern comprising one or more focal spot positions on the at least one target 144. The one or more x-ray focal spot positions on the at least one target 144 may be selected in order to determine the edges of the object 134.

The scouting procedure may further comprise constructing the bounding box 148 around the object 134 (denoted by reference number 122) for each viewing angle of the plurality of differing viewing angles. The known 3D spatial extend of the object may be approximated by using the bounding box 148.

The computed tomography device 150 may have a variable geometry, preferably such that a distance between the object 134 and the x-ray radiation source 131 and/or the distance between the object 134 and the detector 136, may be variable.

The scouting procedure may further comprise adjusting the zoom of the computed tomography device 150 (denoted by reference number 124). Preferably, the zoom may be adjusted by adjusting the distance between the object 134 and the x-ray radiation source 131 and/or adjusting the distance between the object 134 and the detector 136, in a manner that the portion of the object 134 covered by the x-ray cone 132 increases. Preferably, the portion of the object 134 covered by the x-ray cone 132 may increase such that a single projection image may completely capture the object 134 so that only one focal spot scouting position may be required in order to determine the edges of the object 134 and to construct the bounding box 148.

The method may further comprise determining a maximum distance between x-ray focal spot positions (denoted by reference number 126) on the at least one target 144 in the imaging focal spot pattern such that the complete volume of the object 134 to be reconstructed may be irradiated by the different x-ray radiation cones 132 when a PSS is performed Therein, determining a maximum distance may be performed by evaluating the known 3D spatial extend of the object and the relative positions of the x-ray radiation source 131, the object 134 and the detector 136. Determining the maximum distance between x-ray focal spot positions (denoted by reference number 126) may be performed in order to determine the variable x-ray focal spot positions (denoted by reference number 130). Consequently, determining the variable x-ray focal spot positions (denoted by reference number 130) may comprise determining the maximum distance between x-ray focal spot positions (denoted by reference number 126).

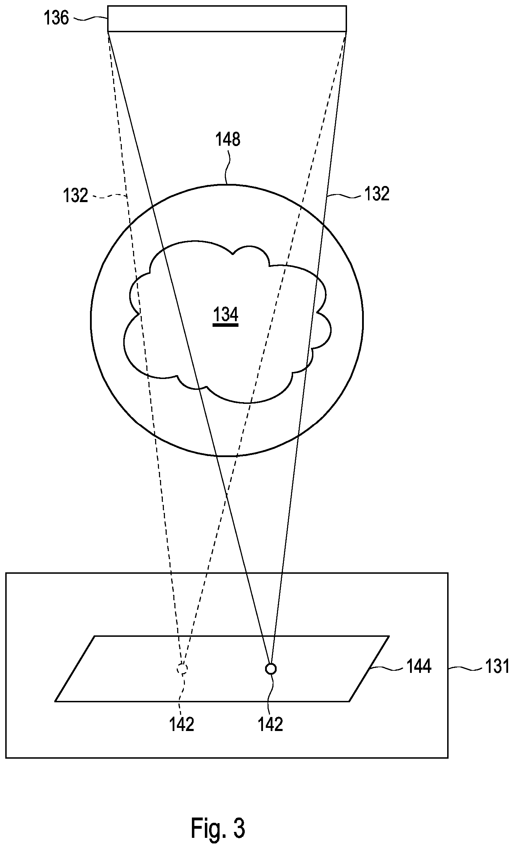

The step of determining the maximum distance between x-ray focal spot positions 142 (denoted by reference number 126) is exemplarily illustrated in Figure 3. Figure 3 shows an object 134 that is again approximated by a bounding box 148. In this exemplary case, the object 134 cannot be fully covered by two x-ray radiation cones 132. It consequently requires more than two x-ray radiation cones 132. In such a case an imaging focal spot pattern may be determined in which the number of x-ray radiation cones 132 required to fully illuminate the object 134 or its approximating bounding box 148 is minimized. This may require that the maximum distance between x-ray focal spot positions 142 is determined.

As already denoted, the maximum distance between x-ray focal spot positions 142 may be determined by evaluating the known 3D spatial extend of the object 134, which may be approximated by using a bounding box 148, and the relative positions of the x-ray radiation source 131, the object 134 and the detector 136. As may be derived from Figure 3, the intersection of two x-ray radiation cones 132 emerging from x-ray focal spot positions 142 should not occur within the object 134 but rather outside of or on an edge of the object 134.

The method may further comprise determining a minimum number of x-ray focal spot positions (denoted by reference number 128) on the at least one target required in the imaging focal spot pattern such that the complete object volume to be reconstructed is irradiated when a PSS is performed. Therein, determining a minimum number of x-ray focal spot positions may be performed by evaluating the known 3D spatial extend of the object and the relative positions of the x-ray radiation source 131, the object 134 and the detector 136. Determining a minimum number of x-ray focal spot positions (denoted by reference number 128) may be performed in order to determine the variable x-ray focal spot positions (denoted by reference number 130). Consequently, determining the variable x-ray focal spot positions (denoted by reference number 130) may comprise determining a minimum number of x-ray focal spot positions (denoted by reference number 128).

The maximum distance between x-ray focal spot positions on the at least one target in the imaging focal spot pattern and the minimum number of x-ray focal spot positions in the imaging focal spot pattern may be determined for each viewing angle of the differing viewing angles of the TSS.

The imaging focal spot pattern may degenerate into a horizontal line pattern in case only a trans-axial field of view of the detector needs to be extended. Alternatively, the imaging focal spot pattern may degenerate into a vertical line pattern in case only an axial field of view of the detector needs to be increased.

Determining the 3D-image of the object 134 by using the image reconstruction algorithm may comprise evaluating each captured projection image and, for each projection image, using the x-ray focal spot position on the at least one target 144 at which the respective projection image has been captured. The image reconstruction algorithm may be or may comprise at least one iterative reconstruction algorithm. Further, the image reconstruction algorithm may be or may comprise at least one analytical reconstruction algorithm.

### List of reference numbers

- 110: method for operating a computed tomography device
- 112: performing a projection image scanning sequence (PSS)
- 114: performing a tomographic scanning sequence (TSS)
- 116: performing an image reconstruction algorithm
- 118: determining the 3D spatial extend of an object
- 120: performing a PSS at a plurality of viewing angles
- 122: constructing the bounding box
- 124: adjusting the zoom of a CT device
- 126: determining the maximum distance between x-ray focal spot positions
- 128: determining a minimum number of x-ray focal spot positions
- 130: determining the variable x-ray focal spot positions
- 131: x-ray radiation source
- 132: x-ray radiation cone
- 134: object
- 136: detector
- 138: electron optic arrangement
- 140: electron beam
- 142: focal spot
- 144: target
- 146: electron gun
- 148: bounding box
- 150: computed tomography device
- 152: control unit

## Claims

1. A method for operating a computed tomography device (150), wherein the computed tomography device (150) comprises at least one x-ray radiation source (131) configured for generating x-ray radiation cones (132) for irradiating an object (134), wherein the computed tomography device (150) comprises at least one detector (136) configured for detecting the x-ray radiation of the x-ray radiation cones (132) that have interacted with the object (134), wherein the x-ray radiation source (131) comprises at least one electron optic arrangement (138) configured for variably arranging at least one electron beam (140) onto a plurality of positions of focal spots on at least one target (144) comprised by the x-ray radiation source (131) in a manner that a plurality of different x-ray radiation cones (132) emerging from the focal spots is generated in order to image different portions of the object (134); the method comprising:
- performing a projection image scanning sequence (PSS) by using an imaging focal spot pattern in order to generate a plurality of projection images, wherein the imaging focal spot pattern comprises a plurality of x-ray focal spot positions on the at least one target (144) such that a volume of the object (134) to be reconstructed is irradiated by the different x-ray radiation cones (132);
- performing a tomographic scanning sequence (TSS) by performing the projection image scanning sequence (PSS) at differing viewing angles;
- performing an image reconstruction algorithm using the projection images in order to reconstruct a 3D-image of the object (134).

2. The method according to the preceding claim, wherein the imaging focal spot pattern is variable in a manner that the x-ray focal spot positions on the at least one target (144) are variable.

3. The method according to the preceding claim, further comprising:
- determining the variable x-ray focal spot positions on the at least one target (144) by using a known 3D spatial extend of the object, preferably wherein the known 3D spatial extend is approximated by using a bounding box (148) enclosing the object (134), wherein the variable x-ray focal spot positions are determined in order to increase the field of view of the detector (136) to cover the complete volume of the object (136) to be reconstructed.

4. The method according to the preceding claim, further comprising:
- determining the known 3D spatial extend of the object by at least one of:
∘ performing a scouting procedure;
∘ receiving a user-input related to the known 3D spatial extend of the object;
∘ receiving data related to the 3D spatial extend of the object, wherein the data related to the 3D spatial extend of the object is selected from at least one of:
▪ data of a model of the object (134); such as a CAD model of the object;
▪ data of a 3D volume representation of the object (134), such as existing 3D tomographic data or volumetric data;

5. The method according to the preceding claim, wherein the determined known 3D spatial extend of the object is an approximated 3D spatial extend of the object, particularly wherein the approximated 3D spatial extend of the object comprises at least one simplified geometric object, such as a cube, a cylinder, a sphere, a cone.

6. The method according to the preceding claim, wherein the scouting procedure comprises
- performing a PSS at a plurality of differing viewing angles, wherein the PSS is using a scout scan pattern comprising one or more focal spot positions on the at least one target (144), wherein the one or more x-ray focal spot positions on the at least one target (144) are selected in order to determine the edges of the object (136).

7. The method according to the preceding claim, wherein the scouting procedure further comprises
- constructing the bounding box (148) around the object (134) for each viewing angle of the plurality of differing viewing angles, wherein the known 3D spatial extend of the object is approximated by using the bounding box (148).

8. The method according to the preceding claim, wherein the computed tomography device (150) has a variable geometry, preferably such that a distance between the object (134) and the x-ray radiation source (131) and/or the distance between the object (134) and the detector (136), is variable, wherein the scouting procedure further comprises
- adjusting the zoom of the computed tomography device (150), preferably by adjusting the distance between the object (134) and the x-ray radiation source (131) and/or adjusting the distance between the object (134) and the detector (136), in a manner that the portion of the object (134) covered by the x-ray cone (132) increases, preferably such that a single projection image completely captures the object (134) so that only one focal spot scouting position is required in order to determine the edges of the object (134) and to construct the bounding box (148).

9. The method according to any one of the five preceding claims, further comprising:
- determining a maximum distance between x-ray focal spot positions on the at least one target (144) in the imaging focal spot pattern such that the complete volume of the object (134) to be reconstructed is irradiated by the different x-ray radiation cones (132) when a PSS is performed, wherein determining a maximum distance is performed by evaluating the known 3D spatial extend of the object and the relative positions of the x-ray radiation source (131), the object (134) and the detector (136).

10. The method according to any one of the six preceding claims, further comprising:
- determining a minimum number of x-ray focal spot positions on the at least one target (144) required in the imaging focal spot pattern such that the complete object volume to be reconstructed is irradiated when a PSS is performed, wherein determining a minimum number of x-ray focal spot positions is performed by evaluating the known 3D spatial extend of the object and the relative positions of the x-ray radiation source (131), the object (134) and the detector (136).

11. The method according to the preceding claims, where the maximum distance between x-ray focal spot positions on the at least one target (144) in the imaging focal spot pattern and the minimum number of x-ray focal spot positions in the imaging focal spot pattern is determined for each viewing angle of the differing viewing angles of the TSS.

12. The method according to any one of the preceding claims, wherein determining the 3D-image of the object by using the image reconstruction algorithm comprises evaluating each captured projection image and, for each projection image, using the x-ray focal spot position on the at least one target (144) at which the respective projection image has been captured.

13. A computed tomography device (150), wherein the computed tomography device (150) comprises at least one x-ray radiation source (131) configured for generating x-ray radiation cones (132) for irradiating an object (134), wherein the computed tomography device (150) comprises at least one detector (136) configured for detecting the x-ray radiation of the x-ray radiation cones (132) that have interacted with the object (134), wherein the x-ray radiation source (131) comprises at least one electron optic arrangement (138) configured for variably arranging at least one electron beam (140) onto a plurality of positions of focal spots on at least one target (144) comprised by the x-ray radiation source (131) in a manner that a plurality of different x-ray radiation cones (132) emerging from the focal spots is generated in order to image different portions of the object (134), wherein the computed tomography device (150) is configured for performing the method according to any one of the preceding claims.

14. A computer program comprising instructions which, when the instructions are executed by the computed tomography device (150) according to the claim referring to the computed tomography device (150), cause the computed tomography device (150) to perform the method according to any one of the preceding claims referring to a method.

15. A computer-readable storage medium, specifically a non-transient computer-readable medium, comprising instructions which, when the instructions are executed by the computed tomography device (150) according to the claim referring to the computed tomography device (150), cause the computed tomography device (150) to perform the method according to any one of the preceding claims referring to a method.
